# EUROPEAN PATENT APPLICATION

(11) **EP 4 696 713 A1**
(43) Date of publication of application: **18.02.2026**
(21) Application number: 25188124.9
(22) Date of filing: 08.07.2025
(51) Int. Cl.: C07K 16/28, C07K 14/705, A61K 35/00, A61P 25/28

(54) **TARGETING VEHICLES, COMPOSITIONS AND USES THEREOF**

(30) Priority: 09.07.2024 US 202463669086 P
(71) Applicant: China Medical University, Taichung City 406040 (TW)
(72) Inventor: Cho, Der-Yang, 404022 Taichung City (TW); Chiu, Shao-Chih, 404022 Taichung City (TW); Chen, Yi-Wen, 404022 Taichung City (TW); Shie, Ming-You, 404022 Taichung City (TW); Huang, Shi-Wei, 404022 Taichung City (TW); Pan, Chih-Ming, 404022 Taichung City (TW); Chen, Cheng-Yu, 404022 Taichung City (TW); Chen, Yen, 540009 Nantou City (TW)
(74) Representative: Cabinet Chaillot

(57) **Abstract**

A targeting vehicles comprises an extracellular vesicle with a dopamine transporter antibody on a transmembrane protein of the extracellular vesicle, the extracellular vesicle is secreted by a cell transfected with a vector gene, and at least a portion of the vector gene comprises SEQ ID No: 1. The targeting vehicles provided in the present invention can be loaded with drugs and cross the blood-brain barrier to achieve specific binding to dopamine neuron, and regulate the secretion of Parkinson's disease marker proteins and delay the course of Parkinson's disease.

## Description

### FIELD OF INVENTION

The present invention relates to a targeting vehicle, a pharmaceutical composition comprising the targeting vehicle, and uses thereof.

### BACKGROUND OF THE INVENTION

Parkinson's disease (PD) is a neurodegenerative disorder of the central nervous system (CNS), commonly affecting the brain. The symptoms of PD typically develop progressively over time, primarily impairing the motor nervous system, and the disease remains incurable. Conventionally, administration of high doses of dopamine drugs, such as L-3,4-dihydroxyphenylalanine (L-DOPA), has been employed to delay disease progression. However, as neurodegeneration advances and neuronal loss accumulates, in combination with the restrictive nature of the blood-brain barrier, the therapeutic efficacy of dopamine drugs diminishes over time. Furthermore, non-specific delivery of large quantities of dopamine drugs to the CNS may inevitably interfere with physiological mechanisms in other regions of the brain. Accordingly, the development of alternative therapeutic strategies to mitigate Parkinson's disease represents a critical and urgent objective in the field.

### SUMMARY OF THE INVENTION

In order to develop technologies capable of mitigating Parkinson's disease, the present invention provides a targeting vehicle comprising an extracellular vesicle, wherein a transmembrane protein of the extracellular vesicle is conjugated with a dopamine transporter antibody, and wherein a protein sequence of the dopamine transporter antibody comprises SEQ ID NO: 4.

The extracellular vesicle is secreted by a genetically modified human embryonic kidney 293 (HEK-293) cell line, wherein a gene sequence of the genetically modified HEK-293 cell line comprises at least a portion of SEQ ID NO: 1.

The targeting vehicle of the present invention can further be loaded with a drug as needed to form a biocompatible pharmaceutical composition.

The drug includes, but is not limited to, gene fragments (DNA, RNA), protein sequences, or chemical agents.

The targeting vehicle of the present invention can be loaded with the drug to cross the blood-brain barrier, achieve specific binding to dopamine neurons, regulate the secretion of Parkinson's disease marker proteins, and provide excellent therapeutic efficacy in delaying the progression of Parkinson's disease.

The present invention further provides a pharmaceutical composition comprising the targeting vehicle, wherein the targeting vehicle is loaded with curcumin.

The pharmaceutical composition comprising the targeting vehicle loaded with curcumin can effectively reduce the accumulation of alpha-synuclein, suppress the expression of inflammation-related proteins including interferon-gamma (INF-γ), interleukin-1 beta (IL-1β), interleukin-6 (IL-6), and tumor necrosis factor-alpha (TNF-α), and upregulate the expression of Parkin protein, DJ-1 protein, tyrosine hydroxylase (TH) protein, and neurogenesis-related proteins including brain-derived neurotrophic factor (BDNF), nerve growth factor (NGF), vascular endothelial growth factor (VEGF), interleukin-10 (IL-10), and ciliary neurotrophic factor (CNTF). Furthermore, the pharmaceutical composition can maintain neural stem cell activity, as indicated by expression of nestin and Ki67 proteins, in the striatum region of the brain. Accordingly, the pharmaceutical composition not only delays the symptomatic progression of Parkinson's disease but also promotes the regeneration and differentiation of dopamine neurons, thereby demonstrating significant therapeutic potential for the treatment of Parkinson's disease.

### BRIEF DESCRIPTION OF THE DRAWINGS

FIG. 1 is a block diagram illustrating steps for a preferred embodiment of the targeting vehicle provided by the present invention.
FIGS. 2A and 2B are protein expression diagrams illustrating results for a first preferred embodiment of the targeting vehicle provided by the present invention.
FIG. 3A is a Transmission electron microscope (TEM) image of a preferred embodiment of the targeting vehicle provided by the present invention.
FIG. 3B is a Western blot diagram illustrating protein expression results for the first preferred embodiment of the targeting vehicle provided by the present invention.
FIGS. 4A and 4B are TEM images of the first and second preferred embodiments of the targeting vehicle after drug loading.
FIGS. 5A and 5B are flow cytometry diagrams illustrating detection results for the first and second preferred embodiments of the targeting vehicle after drug loading.
FIG. 6A is a schematic diagram of a blood-brain barrier cell culture model provided by the present invention.
FIG. 6B is an immunofluorescence staining diagram illustrating results from Experiment 1 provided by the present invention.
FIG. 7A is a diagram illustrating intracellular reactive oxygen species concentration results from Experiment 2 provided by the present invention.
FIG. 7B is a Western blot diagram illustrating protein expression results from Experiment 2 provided by the present invention.
FIG. 7C is a diagram illustrating cell viability results from Experiment 2 provided by the present invention.
FIG. 8 is a diagram illustrating the distribution of the targeting vehicle in a Parkinson's disease animal model provided by the present invention.
FIG. 9 is a rotarod test diagram illustrating results for the Parkinson's disease animal model treated with the first preferred embodiment of the targeting vehicle.
FIGS. 10A to 10C are open field test diagrams illustrating results for the Parkinson's disease animal model treated with the first preferred embodiment of the targeting vehicle.
FIGS. 11A to 11C are diagrams illustrating brain slice and blood analysis results for the Parkinson's disease animal model treated with the first preferred embodiment of the targeting vehicle.
FIGS. 12A and 12B are rotarod test and open field test diagrams illustrating results for the Parkinson's disease animal model treated with the second preferred embodiment of the targeting vehicle.
FIG. 12C is a brain slice analysis diagram illustrating results for the Parkinson's disease animal model treated with the second preferred embodiment of the targeting vehicle.
FIGS. 13A and 13B are protein expression diagrams illustrating results for human PD disease iPSC-derived neuron cells treated with the first preferred embodiment of the targeting vehicle.
FIG. 14A is a diagram illustrating an intracellular protein concentration result from Experiment 7 provided by the present invention.
FIG. 14B is a Western blot diagram illustrating protein expression results from Experiment 7 provided by the present invention.
FIGS. 15A and 15B are rotarod test and open field test diagrams illustrating results for the Parkinson's disease animal model treated with the third preferred embodiment of the targeting vehicle.
FIG. 15C is a protein expression analysis diagram in the brain illustrating results for the Parkinson's disease animal model treated with the third preferred embodiment of the targeting vehicle.

### DETAILED DESCRIPTION OF THE PREFERRED EMBODIMENTS

A known pathological hallmark of Parkinson's disease (PD) is the degeneration and death of dopamine neurons that project from the substantia nigra to the striatum region of the brain, wherein an expression level of a dopamine transporter (DAT), which is expressed on the dopamine neurons in the striatum, may serve as an indicator for evaluating the progression of Parkinson's disease.

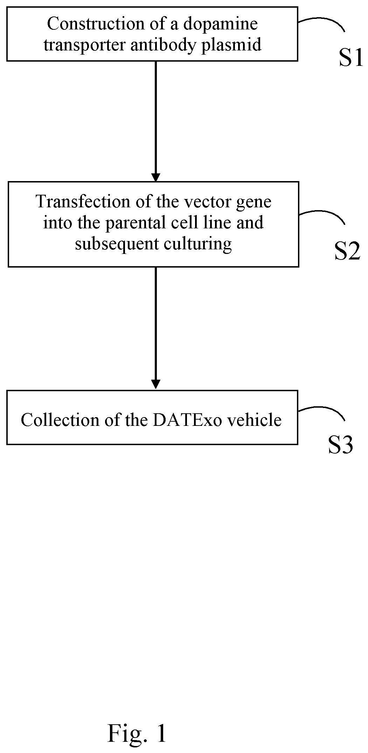

Referring to FIG. 1, in order to achieve specific therapeutic effects, the present invention genetically modifies a parental cell line to enable an extracellular vesicle secreted by the parental cell line to express a dopamine transporter antibody (hereinafter referred to as "anti-DAT") on a surface thereof, thereby constructing a targeted extracellular vesicle (hereinafter referred to as "DATEV vehicle"). The DATEV vehicle is thereby capable of specifically binding to dopamine neurons that express the dopamine transporter (DAT) through the interaction with the anti-DAT, thereby promoting phagocytosis by the dopamine neurons while minimizing nonspecific binding to normal cells.

The anti-DAT is preferably expressed on a transmembrane protein of the DATEV vehicle, such as CD63, CD81, or CD9. In the present embodiment, the anti-DAT is expressed on a CD63 transmembrane protein of the DATEV vehicle.

In the present embodiment, preparation of the DATEV vehicle comprises the following steps:
**Step S1: Construction of a dopamine transporter antibody plasmid.** A gene fragment encoding the dopamine transporter antibody is prepared, and a nucleic acid sequence of the dopamine transporter antibody is inserted into a target gene of the transmembrane protein by means of genetic engineering, such that the gene fragment and the target gene are combined to form a vector gene. In the present embodiment, the target gene SEQ ID NO: 3, which encodes the CD63 transmembrane protein of the extracellular vesicle, is used for illustration.

The gene fragment comprises SEQ ID NO: 1 or SEQ ID NO: 2.

The gene fragment is inserted into a gene sequence corresponding to an extracellular loop located between the third and fourth transmembrane domains of the transmembrane protein.

In one embodiment, the gene fragment comprising SEQ ID NO: 1 or SEQ ID NO: 2 is inserted at amino acid position 135 of the target gene SEQ ID NO: 3 or SEQ ID NO: 5 to form the vector gene.

In one embodiment, insertion of the gene fragment comprising SEQ ID NO: 2 into the target gene SEQ ID NO: 3 results in the formation of the vector gene SEQ ID NO: 4.

In one embodiment, insertion of the gene fragment comprising SEQ ID NO: 1 into the target gene SEQ ID NO: 5 results in the formation of the vector gene SEQ ID NO: 6.

The vector gene SEQ ID NO: 4 is subsequently recombined into a plasmid using gene cloning techniques. In the present embodiment, the plasmid comprises a pEXO plasmid (Addgene, Watertown, MA, USA).

**Step S2: Transfection of the vector gene into the parental cell line and subsequent culturing.** The plasmid carrying the vector gene SEQ ID NO: 4 is introduced into the parental cell line by means of transfection, thereby producing a DAT cell line capable of highly expressing the anti-DAT. The transfection techniques may include electroporation, cell squeezing, ultrasound, viral transfection, or chemical transfection, etc. In the present embodiment, liposome-mediated transfection (Lipofectamine 3000, L3000015, Invitrogen, Waltham, MA, USA) is employed.

The selection of the parental cell line is not particularly limited and may be determined based on the characteristics of the selected cell type. For example, a human embryonic kidney 293 (HEK-293) cell line may be employed due to its high transfection efficiency, rapid growth rate, ease of culturing, and capacity to produce large quantities of cells and secreted extracellular vesicles in a relatively short time. Alternatively, mesenchymal stem cells (MSCs), which are rich in growth factors and anti-inflammatory factors, may be used so that the extracellular vesicles secreted therefrom inherently carry growth-promoting and anti-inflammatory factors capable of producing direct therapeutic effects.

A total of 2×10⁸ DAT-293T cells are seeded into a culture vessel (CelCradle^{®} benchtop bioreactor, ESCO Aster, Singapore) and cultured in 500 mL of Dulbecco's Modified Eagle's Medium (DMEM), supplemented with extracellular vesicle-depleted fetal bovine serum (FBS; Gibco, Grand Island, NY, USA) and 1% antibiotics (penicillin/streptomycin/amphotericin B solution).

Step S3: Collection of the DATEV vehicle. Following culture of the DAT-293T cells for 3 to 4 days, the culture medium is collected and passed through a 0.22 µm filter. The resulting filtrate is concentrated and purified using a tangential flow filtration system (MAP.03-plus TFF System, Lefo Science) equipped with a 300 kDa molecular weight cutoff membrane. The supernatant is subsequently subjected to chromatographic elution, further concentrated using a 30 kDa molecular weight cutoff membrane, and finally resuspended in phosphate-buffered saline (PBS), thereby obtaining the DATEV vehicle released into the culture medium.

Through the foregoing procedures, a first embodiment and a second embodiment are established, wherein the gene fragment comprising SEQ ID NO: 1 or SEQ ID NO: 2 is transfected into the DAT-293T cell line, respectively, to produce the DATEV vehicle. The affinity of the DATEV vehicle obtained in the first embodiment and the second embodiment ranges from KD = 1.3 to 6.4 × 10⁻⁹ M.

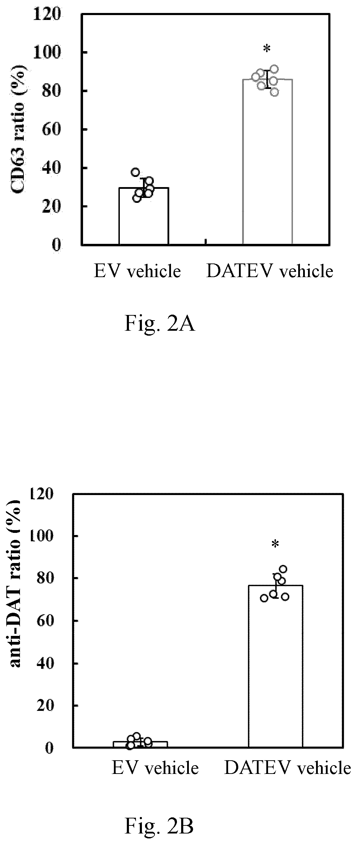

In FIGS. 2A and 2B, the expression levels of CD63 and anti-DAT on the DATEV vehicle of the first embodiment are quantitatively analyzed using antibody-antigen techniques and compared to a general extracellular vesicle (hereinafter referred to as "EV vehicle") secreted by a non-transfected HEK-293 cell line, in order to confirm differences in the expression levels of the transmembrane protein CD63 and anti-DAT. The culturing and collection procedures for the general HEK-293 cells and the EV vehicle are conducted under the same conditions as those used for the DAT-293T cells and the DATEV vehicle.

After collection, the DATEV vehicle and the EV vehicle are incubated for 6 to 12 hours in a reaction solution containing fluorescently labeled CD63 antibodies and fluorescently labeled recombinant DAT protein. The reaction solution is subsequently replaced with a buffer solution (PBS-T containing 0.1% casein), and 50 µL of the reaction mixture is dispensed into each well of a detection plate. An automated extracellular vesicle absolute quantification analyzer (ExoCounter, JVCKENWOOD Corporation, Yokosuka, Japan) is employed to determine the proportions of DATEV vehicles and EV vehicles exhibiting surface expression of CD63 and/or anti-DAT. The results indicate that the DATEV vehicle obtained from the DAT-293T cell line exhibits significantly higher expression levels of the transmembrane protein CD63 and anti-DAT relative to the EV vehicle obtained from the non-transfected HEK-293 cell line. In the second embodiment, the expression levels of CD63 and anti-DAT on the DATEV vehicle also reach as high as 94% and 91%, respectively (data not shown).

The present invention also provides a third preferred embodiment different from the second preferred embodiment in that applying the mesenchymal stem cell (MSC) as the parental cell line, transfecting the MSC cell via lentivirus transfection and forming a DAT-MSC cell line after transfection. Wherein, the MSC cells are cultured in 20 mL of a culture medium containing fetal bovine serum (FBS), and the culture medium containing fetal bovine serum (FBS) is supplemented with 8 µg/mL of polybrene (PB), 50 mg/mL of protamine sulfate (PS), and 1100 mg/mL of Synperonic F108 (F108). Furthermore, a multiplicity of infection (MOI) of the MSC cell is greater than 2 (virus number/cell number) when lentivirus transfection.

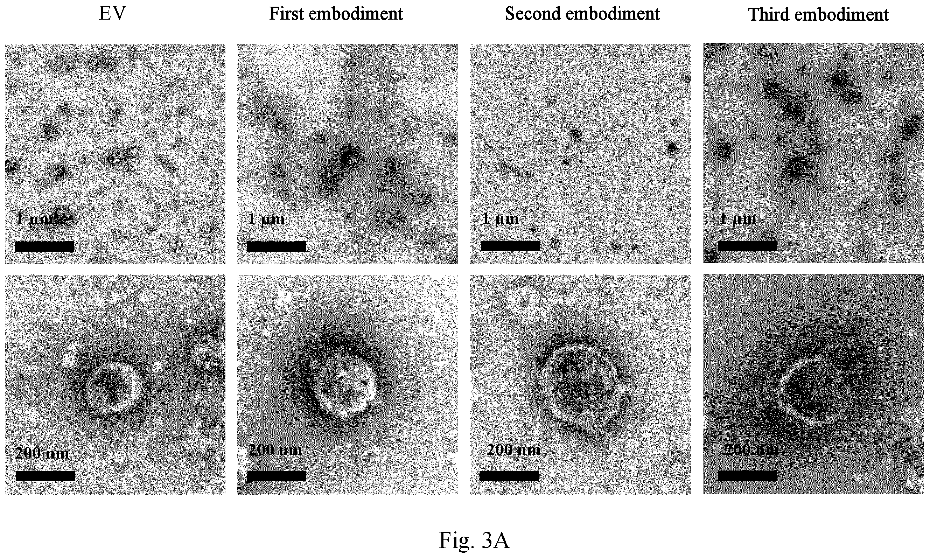

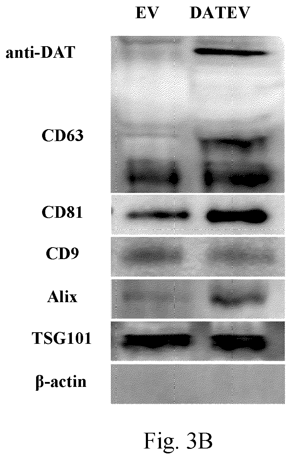

As shown in FIG. 3A, transmission electron microscope (TEM) reveals that, in all the first, the second, and the third embodiment, the DATEV vehicle expresses anti-DAT while maintaining overall size and morphology comparable to the EV vehicle. FIG. 3B presents Western blot results confirming differences in protein expression between the DATEV vehicle and the EV vehicle. Specifically, the DATEV vehicle exhibits higher expression levels of transmembrane proteins, particularly CD63 and CD81, as well as anti-DAT, relative to the EV vehicle. These results confirm that the DAT-293T cell line, following gene transfection and expression of anti-DAT, secretes the DATEV vehicle exhibiting surface expression of anti-DAT.

The targeting vehicle provided by the present invention may further be loaded with a drug, as necessary, to form a biocompatible pharmaceutical composition. The drug includes, but is not limited to, gene fragments (DNA, RNA), protein sequences, or chemical agents.

Curcumin exhibits excellent therapeutic properties, having been demonstrated to promote wound healing, exert anti-cancer effects, provide anti-COVID-19 activity, and regulate the immune system. In addition, numerous studies have shown that curcumin can effectively suppress the expression of neurodegenerative factors, such as phosphorylated tau protein and β-amyloid precursor protein, thereby indicating its potential utility in the treatment of Parkinson's disease.

Brain-derived neurotrophic factor (BDNF) is a critical bioactive protein in the brain, involved in regulating neuronal survival, growth, and synaptic plasticity. Numerous studies have demonstrated a strong association between insufficient BDNF expression and the development of neurodegenerative disorders, including Alzheimer's disease and Parkinson's disease.

To verify that the targeting vehicle provided by the present invention possesses targeting, drug-loading, and drug-release capabilities, the DATEV vehicle is loaded with curcumin or with messenger ribonucleic acid (mRNA) encoding BDNF (mRNA-BDNF-CY3) to form a curcumin-loaded DATEV vehicle (hereinafter referred to as "Cur@DATEV") and a BDNF-loaded DATEV vehicle (hereinafter referred to as "BDNF@DATEV"), respectively, for evaluation of their therapeutic effects in models of Parkinson's disease.

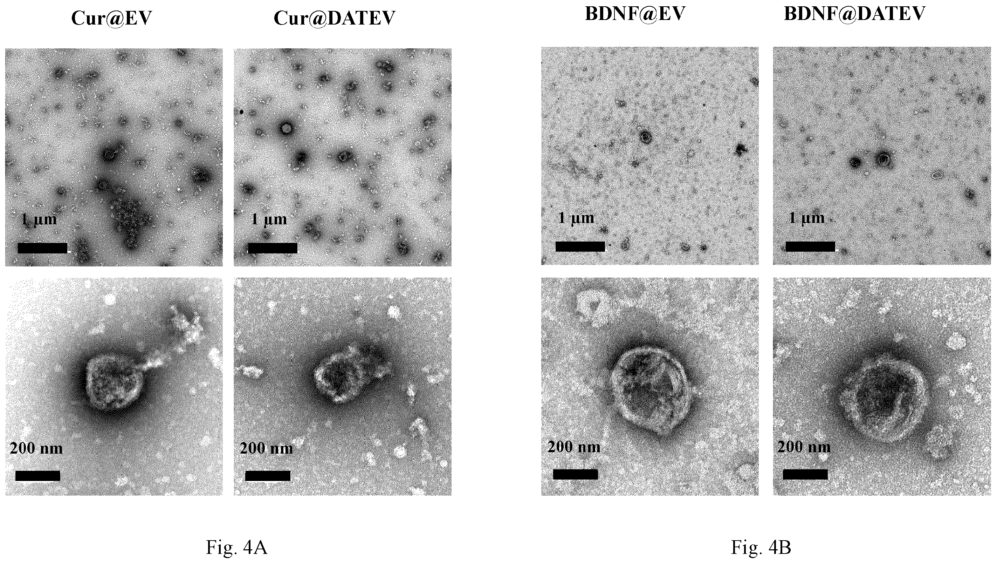

Referring to FIGS. 4A and 4B, the DATEV vehicle and the EV vehicle prepared according to the first embodiment are loaded with curcumin to form Cur@DATEV and a curcumin-loaded EV vehicle (hereinafter referred to as "Cur@EV"), respectively. These preparations are then subjected to subsequent cell-based and animal experiments to evaluate the targeting specificity, functional efficacy, and therapeutic potential of curcumin in treating Parkinson's disease. Similarly, the DATEV vehicle prepared according to the second embodiment is loaded with mRNA-BDNF-CY3 to form BDNF@DATEV, which is compared with an EV vehicle loaded with mRNA-BDNF-CY3 (hereinafter referred to as "BDNF@EV") to evaluate the targeting specificity, functional efficacy, and therapeutic potential of BDNF modulation in treating Parkinson's disease.

In the present embodiment, the DATEV vehicle and the EV vehicle are loaded with curcumin or mRNA-BDNF-CY3 using either ultrasound-mediated loading or electroporation loading methods.

The procedure for loading curcumin into the DATEV vehicle and the EV vehicle using ultrasound comprises the following steps:

The DATEV vehicle and the EV vehicle are each mixed with human serum albumin at a weight ratio ranging in 1:0.1 to 1:2 to form a mixture. The mixture is then subjected to 1-10 cycles of ultrasonic treatment, each consisting of 30 seconds on and 30 seconds off, with the mixture cooled on ice for 2 minutes between cycles. Thereafter, the mixture is filtered through a first filtration membrane having a molecular weight cutoff of 100 kDa. Curcumin is subsequently added to the filtered mixture at a weight ratio of 1:1:1 (µg) relative to the amounts of human serum albumin, DATEV vehicle or EV vehicle, and curcumin. The mixture is then subjected to six additional ultrasound cycles under the same conditions. Following ultrasonic processing, the mixture is filtered twice using a second filtration membrane having a molecular weight cutoff of 30 kDa and resuspended in phosphate-buffered saline (PBS) for subsequent experimental use.

The procedure for loading mRNA-BDNF-CY3 into the DATEV vehicle and the EV vehicle using electroporation comprises the following steps:

The DATEV vehicle or the EV vehicle is mixed with mRNA-BDNF-CY3 and then subjected to electroporation under conditions comprising an applied voltage of 100 to 250 V, a pulse duration of 50 to 300 µs, a pulse interval of 500 to 1500 µs, and 4 to 6 discharge cycles. Thereafter, the mixture is incubated in a cell culture incubator for 30 to 90 minutes, centrifuged, and resuspended in PBS for subsequent experimental use.

As shown in FIG. 4A, transmission electron microscope (TEM) demonstrates that Cur@DATEV, formed by loading curcumin into the DATEV vehicle, maintains an overall size and morphology comparable to that of Cur@EV. Similarly, as shown in FIG. 4B, the size and morphology of BDNF@DATEV and BDNF@EV remain comparable, thereby confirming that conjugation of anti-DAT to the surface of the DATEV vehicle does not impair its drug-loading capacity.

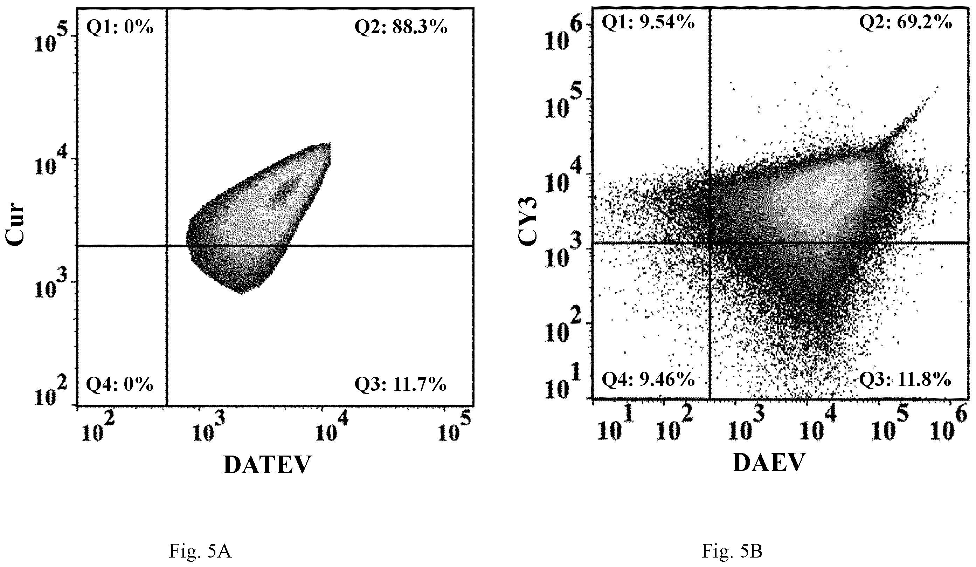

FIGS. 5A and 5B illustrate flow cytometry detection results for the first and second embodiments following drug loading with curcumin and mRNA-BDNF-CY3, respectively. The flow cytometer detects Cur@DATEV based on the intrinsic fluorescence of curcumin, and detects both Cur@DATEV and BDNF@DATEV based on the fluorescence of CY3 carried by mRNA-BDNF-CY3. Based on statistical conversion of the detection data, as shown in the Q2 quadrant results of FIGS. 5A and 5B, approximately 88.3% of the DATEV vehicle prepared according to the first embodiment successfully incorporates curcumin to form Cur@DATEV, and approximately 69.2% of the DATEV vehicle prepared according to the second embodiment successfully incorporates mRNA-BDNF-CY3 to form BDNF@DATEV.

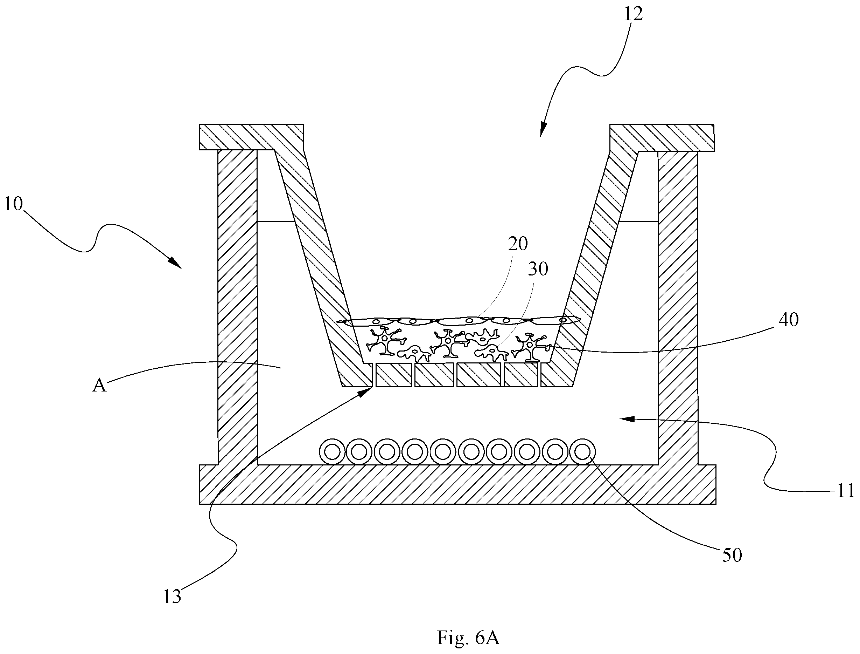

Referring to FIG. 6A, in order to verify that the DATEV vehicle and/or Cur@DATEV are capable of functioning in vivo, a blood-brain barrier cell culture model 10 is established to simulate the physiological environment of the blood-brain barrier. This model enables evaluation of the ability of the drug vehicle to traverse the blood-brain barrier and to bind specifically to a Parkinson's disease model cell line. The Parkinson's disease model cell line is derived from the differentiation of a human neuroblastoma cell line (hereinafter referred to as "SH-SY5Y cell line"), which expresses DAT protein and is commonly utilized in Parkinson's disease research.

The SH-SY5Y cells are cultured in a mixed medium composed of Minimum Essential Medium (MEM; Invitrogen) and Ham's F-12 Nutrient Mix (F-12; Thermo Fisher) in a 1:1 volume ratio, supplemented with 10% fetal bovine serum (FBS; US-sourced HyClone, GE), 1% sodium pyruvate (Thermo Fisher), 1% GlutaMAX^{™} supplement, and 1% penicillin-streptomycin (Thermo Fisher). The cells are maintained in a 37°C, 5% CO₂, humidity-controlled environment. Upon reaching a confluency of 70 - 80% of the culture dish area, neuronal differentiation is induced by treating the SH-SY5Y cells with 50 µM retinoic acid (RA) for two days, followed by daily replacement of a differentiation medium containing 50 nM 12-O-tetradecanoylphorbol-13-acetate (TPA) for an additional five days, thereby producing the Parkinson's disease model cell line.

The blood-brain barrier cell culture model 10 comprises a first culture region 11 and a second culture region 12, which are fluidically connected by a plurality of perforations 13. Both the first culture region 11 and the second culture region 12 are filled with a culture medium A. The Parkinson's disease model cell line 50 is cultured at a bottom surface of the first culture region 11, while an endothelial cell 20 layer, a pericyte 30 layer, and an astrocyte 40 layer are sequentially positioned at a bottom surface of the second culture region 12 adjacent to the perforations 13. The blood-brain barrier cell culture model 10 exhibits an electrical resistance of 2144 Ω·cm², thereby effectively simulating the impedance environment of a physiological blood-brain barrier. In subsequent experiments, one of the EV vehicle, the DATEV vehicle, Cur@DATEV, or Cur@EV is introduced into the second culture region 12, and the Parkinson's disease model cell line 50 cultured in the first culture region 11 is analyzed to verify the efficiency and therapeutic effects of drug delivery across the simulated blood-brain barrier.

### Experiment 1

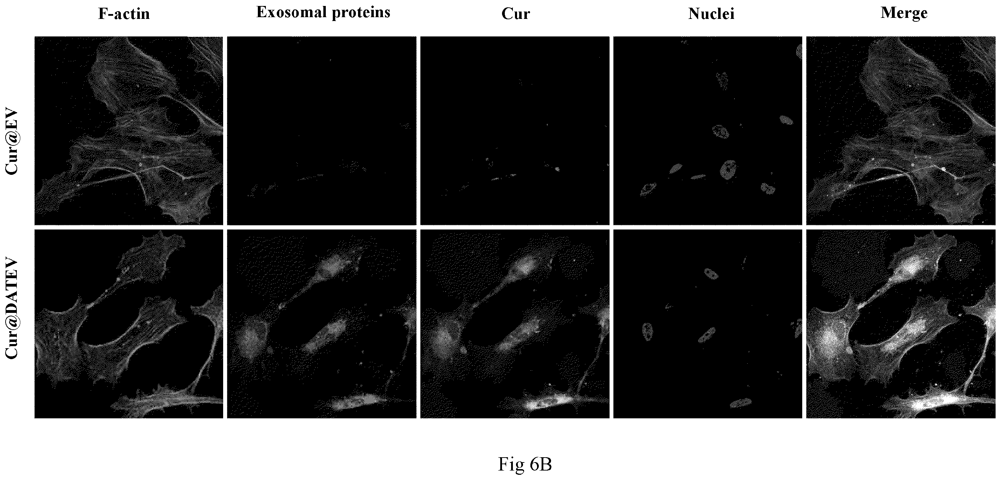

To verify that the DATEV vehicle is capable of traversing the impedance environment of the blood-brain barrier constructed by the blood-brain barrier cell culture model 10 and being phagocytosed by the Parkinson's disease model cell line 50, the DATEV vehicle of the first embodiment is utilized as a representative example as shown in FIG. 6B. In this experiment, Cur@EV is employed as a control group, and Cur@DATEV is employed as an experimental group, in order to compare the phagocytosis of each by the Parkinson's disease model cell line.

The phagocytic efficiency of the Parkinson's disease model cell line was evaluated following incubation with Cur@DATEV and Cur@EV for 24 hours. The intracellular content of curcumin within the Parkinson's disease model cell line was quantified based on the intrinsic fluorescence properties of curcuminoids, thereby allowing comparison of the phagocytosis efficiency of Cur@DATEV and Cur@EV by the Parkinson's disease model cell line. In parallel, an immunofluorescence staining and exosomal protein labeling technique (Protein EV Labeling Kit (Red), ExoGlow^{™}, System Biosciences, Palo Alto, CA, USA) was employed to detect the intracellular presence of Cur@DATEV and Cur@EV. Exosomal proteins were labeled with red fluorescence to confirm internalization of Cur@DATEV and Cur@EV into the Parkinson's disease model cell line via phagocytosis. In addition, F-actin was labeled with purple fluorescence to visualize the cytoskeleton, nuclei were labeled with blue fluorescence, and curcumin was visualized by its green fluorescence, thereby confirming its intracellular delivery by Cur@DATEV and/or Cur@EV.

As shown in FIG. 6B, the experimental group treated with Cur@DATEV exhibits significantly stronger red fluorescence (exosomal proteins) and green fluorescence (curcumin) within the Parkinson's disease model cell line, compared to the control group treated with Cur@EV. These results confirm that the DATEV vehicle exhibits specificity toward the DAT protein and is capable of being internalized into the Parkinson's disease model cell line via phagocytosis.

### Experiment 2

Using the DATEV vehicle of the first embodiment as a representative example, this experiment further evaluates whether Cur@DATEV can traverse the impedance environment of the blood-brain barrier simulated by the blood-brain barrier cell culture model 10, be phagocytosed by the Parkinson's disease model cell line 50, release curcumin intracellularly, and exert therapeutic efficacy. The blood-brain barrier cell culture model 10 is treated under the following conditions:
- A first control group (PD), receiving no treatment;
- A second control group (EV), treated with the EV vehicle;
- A third control group (DATEV), treated with the DATEV vehicle;
- A fourth control group (Cur), treated with free curcumin;
- A fifth control group (Cur@EV), treated with Cur@EV vehicle; and
- An experimental group (Cur@DATEV), treated with Cur@DATEV vehicle.

Following phagocytosis by the Parkinson's disease model cell line 50, the release of curcumin and therapeutic efficacy are subsequently evaluated.

After 48 hours of incubation, cellular responses of the Parkinson's disease model cell line 50 are assessed, including: cell mortality rate, intracellular reactive oxygen species (ROS) concentration, and expression levels of Parkinson's disease-related marker proteins. Alpha-synuclein (α-syn) serves as a pathological marker commonly accumulated in dopamine neurons of Parkinson's disease patients, whereas Parkin protein, DJ-1 protein, and tyrosine hydroxylase (TH) protein serve as marker proteins associated with cellular repair responses following dopamine neuron injury.

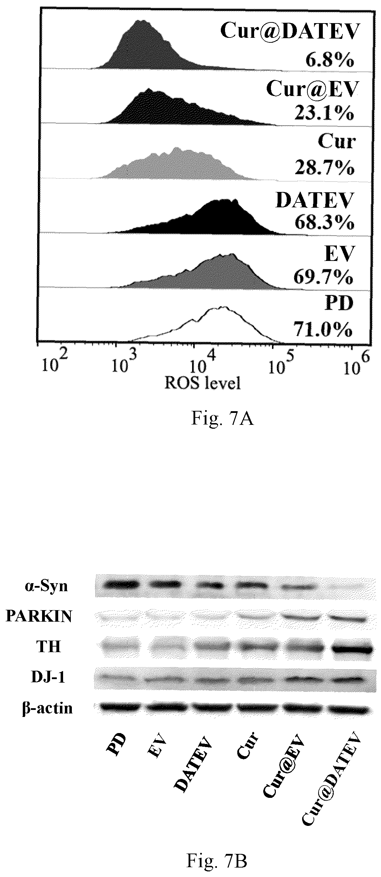

The intracellular ROS concentration of the Parkinson's disease model cell line 50 is determined by flow cytometry analysis. As shown in FIG. 7A, the ROS concentrations measured in the fourth control group, fifth control group, and experimental group are significantly reduced compared to those in the first, second, and third control groups.

As shown in FIG. 7B, Western blot analysis of alpha-synuclein, Parkin protein, DJ-1 protein, and TH protein in the Parkinson's disease model cell line 50 reveals that treatment with Cur@DATEV results in decreased expression of alpha-synuclein and increased expression of Parkin protein, DJ-1 protein, and TH protein, thereby indicating an enhancement of cellular repair capacity.

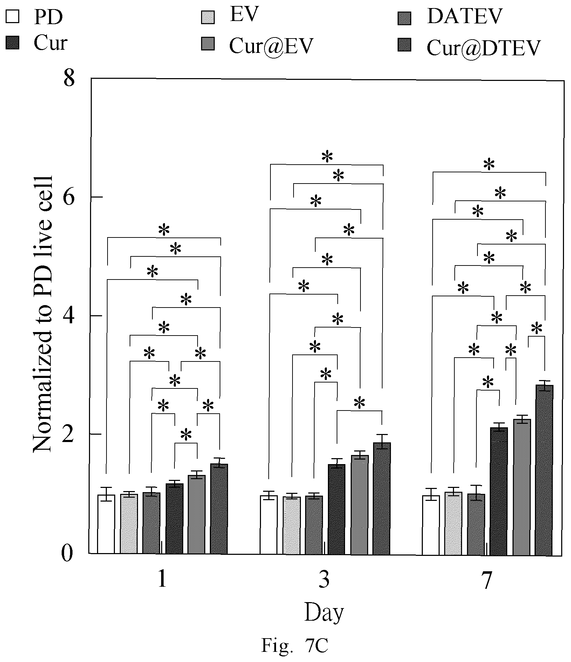

Referring to FIG. 7C, the Parkinson's disease model cell line 50, which represents degenerated dopamine neurons, exhibits low intrinsic repair capacity and is prone to cell death. In this experiment, cell counts for each treatment group are normalized relative to the cell count of the first control group on days 1, 3, and 7 after treatment. Beginning on day 3, the cell counts of the fourth control group, fifth control group, and experimental group are significantly higher than those of the first, second, and third control groups. Notably, by day 7, the experimental group exhibits a significantly higher cell count than both the fourth and fifth control groups, thereby confirming that Cur@DATEV enables intracellular release of curcumin and enhances the therapeutic efficacy of curcumin relative to the fourth and fifth control groups.

To evaluate behavioral changes following treatment with Cur@DATEV and BDNF@DATEV, a Parkinson's disease animal model is established. Rats undergo rotarod training to establish baseline motor performance and are also subjected to open field tests to assess normal behavioral activity. Parkinson's disease symptoms are subsequently induced by stereotactic injection of 6-hydroxydopamine (6-OHDA) into the left dorsal striatum region of the brain to selectively destroy dopamine neurons. Each injection consists of 3.2 µL of 6-OHDA solution containing 11 µg of 6-OHDA powder per unit volume.

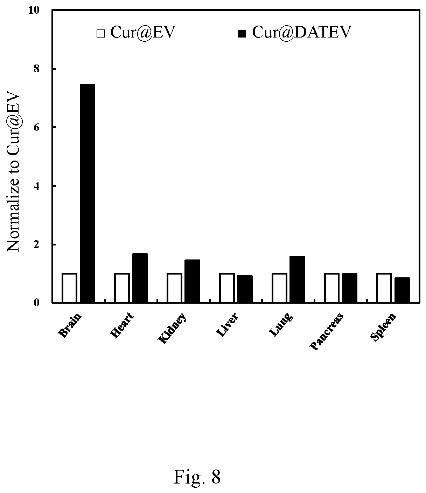

To verify the targeting specificity of the DATEV vehicle in vivo, the DATEV vehicle of the first embodiment is utilized as an example. Cur@DATEV and Cur@EV are administered by intravenous injection after being pre-labeled with a lipophilic fluorescent dye (XenoLight DiR, PerkinElmer) to enable fluorescence detection. Twenty-four hours after administration, an In Vivo Imaging System (IVIS) is used to measure the distribution of Cur@DATEV and Cur@EV across various organs. As shown in FIG. 8, fluorescence intensities are normalized relative to the Cur@EV group, revealing significantly elevated fluorescence intensity in the brain for the Cur@DATEV group, thereby confirming both the targeting specificity of the DATEV vehicle and its ability to traverse the blood-brain barrier. In addition, the DATEV vehicle persists within the brain tissue for up to 7 days (data not shown).

### Experiment 3

After confirming that Cur@DATEV is capable of traversing the blood-brain barrier in the Parkinson's disease animal model, this experiment further investigates whether Cur@DATEV can release curcumin in vivo, alleviate Parkinson's disease symptoms, and achieve therapeutic effects. Beginning on day 0 (i.e., the day of surgery), the Parkinson's disease animal model is treated weekly by intravenous injection for five consecutive weeks, and the animals are assigned to the following groups:
- A healthy control group (Health), which undergoes sham surgery in the dorsal striatum brain area and receives no drug treatment;
- A first control group (PD), which receives no drug treatment;
- A second control group (EV), treated with the EV vehicle;
- A third control group (DATEV), treated with the DATEV vehicle;
- A fourth control group (Cur), treated with free curcumin;
- A fifth control group (Cur@EV), treated with Cur@EV vehicle, wherein both the fourth and fifth control groups receive curcumin at a dosage of 1 mg per 100 g of body weight;
- An experimental group (Cur@DATEV), treated with Cur@DATEV vehicle.

Following treatment, curcumin release and therapeutic efficacy are evaluated.

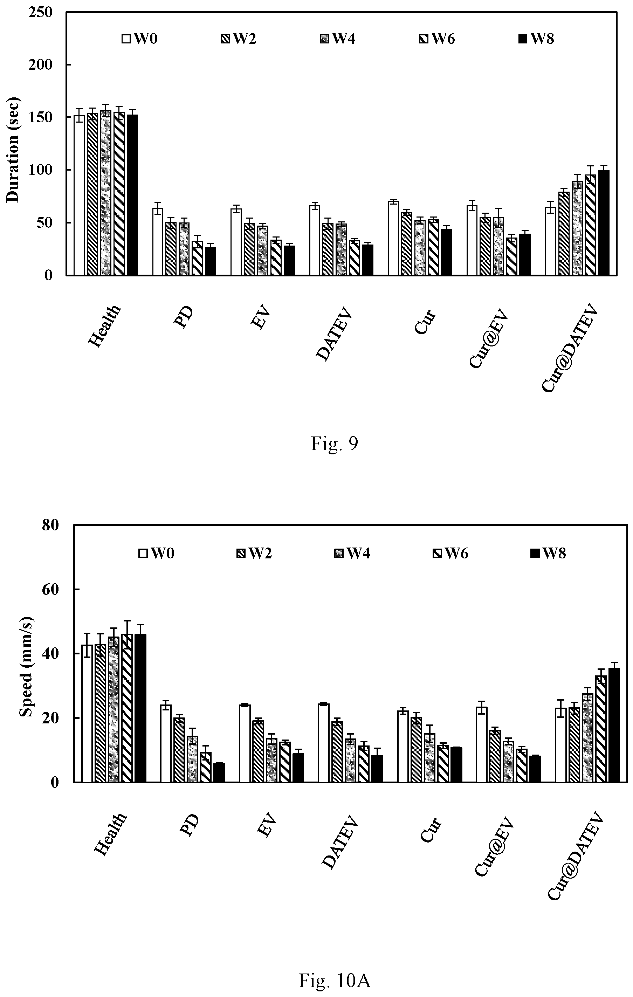

Referring to FIG. 9, rotarod tests are conducted at weeks 0, 2, 4, 6, and 8 to assess motor performance, including the time duration the rats are able to remain on the rotating rod. The results indicate that motor performance in all control groups (i.e., first through fifth control groups) and the experimental group is significantly impaired compared to the healthy control group.

However, relative to the second (EV), third (DATEV), fourth (Cur), and fifth (Cur@EV) control groups, only the experimental group (Cur@DATEV) exhibits progressive improvement in motor performance over time, as a function of increasing cumulative Cur@DATEV administration. Notably, following the final injection at week 5, motor performance in the experimental group continues to improve at weeks 6 and 8, rather than declining, thereby demonstrating sustained therapeutic efficacy.

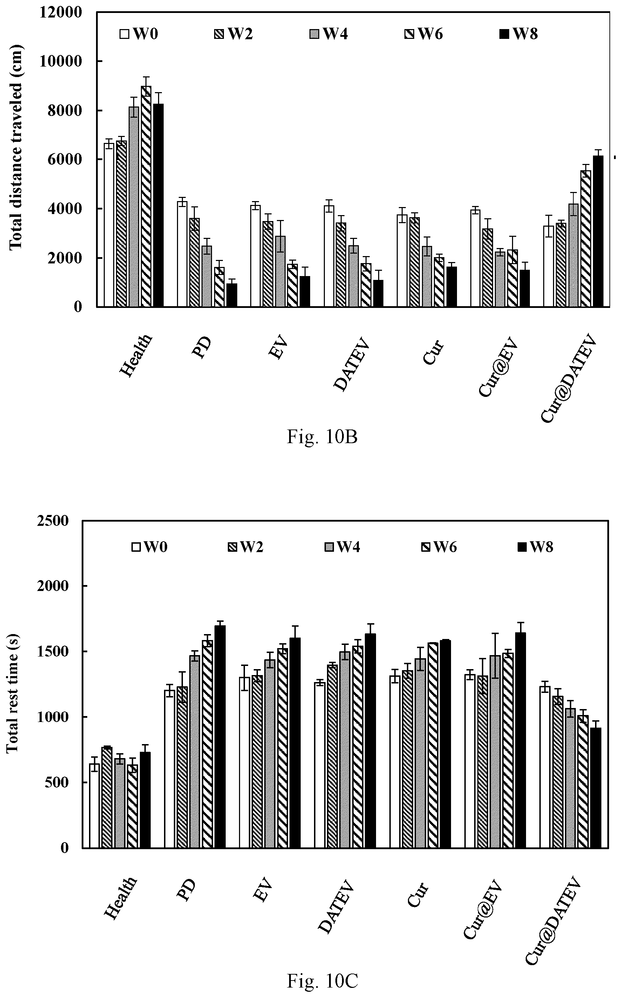

Referring to FIGS. 10A to 10C, open field tests are conducted at weeks 0, 2, 4, 6, and 8 to evaluate motor function, including measurements of movement speed, total distance traveled, and total rest time. Consistent with the rotarod test results, the experimental group (Cur@DATEV) exhibits progressive improvements in motor performance with increasing treatment duration, demonstrating improvements in movement speed, total distance, and reduced rest time. These results confirm that Cur@DATEV not only alleviates motor deficits associated with Parkinson's disease but also restores behavioral function in the Parkinson's disease animal model.

### Experiment 4

Since Experiment 3 confirms that Cur@DATEV improves behavioral performance in the Parkinson's disease animal model, this experiment further analyzes brain tissue and blood samples collected from each group for biochemical evaluation.

Brain slices from the striatum region of each group are prepared, and antibody-antigen binding reactions are used to detect expression of alpha-synuclein (a pathological marker of Parkinson's disease), TH protein (a marker of neuronal repair), and DAT protein (a marker of dopamine neurons).

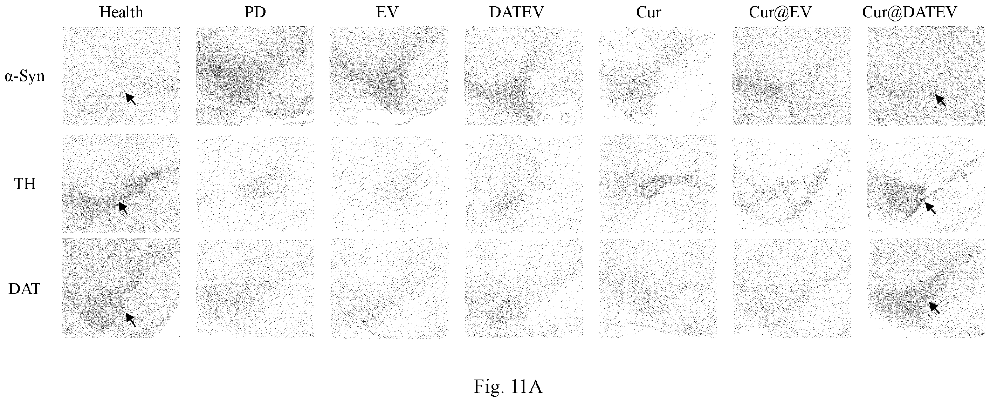

As shown in FIG. 11A, the experimental group (Cur@DATEV) exhibits significantly reduced alpha-synuclein accumulation in the dorsal striatum relative to the first through fifth control groups, accompanied by elevated expression of TH protein and DAT protein. Compared to the healthy control group, the experimental group shows no statistically significant differences in the expression levels of alpha-synuclein, TH protein, or DAT protein.

Blood samples collected from each group are analyzed using antigen-labeling techniques applied to a protein microarray to evaluate the expression levels of neurogenesis-related and inflammation-related proteins.

The neurogenesis-related proteins analyzed include brain-derived neurotrophic factor (BDNF), nerve growth factor (NGF), vascular endothelial growth factor (VEGF), interleukin-10 (IL-10), and ciliary neurotrophic factor (CNTF), while the inflammation-related proteins analyzed include interferon-gamma (INF-γ), interleukin-1 beta (IL-1β), interleukin-6 (IL-6), and tumor necrosis factor-alpha (TNF-α). Protein expression values are normalized relative to the healthy control group for analysis.

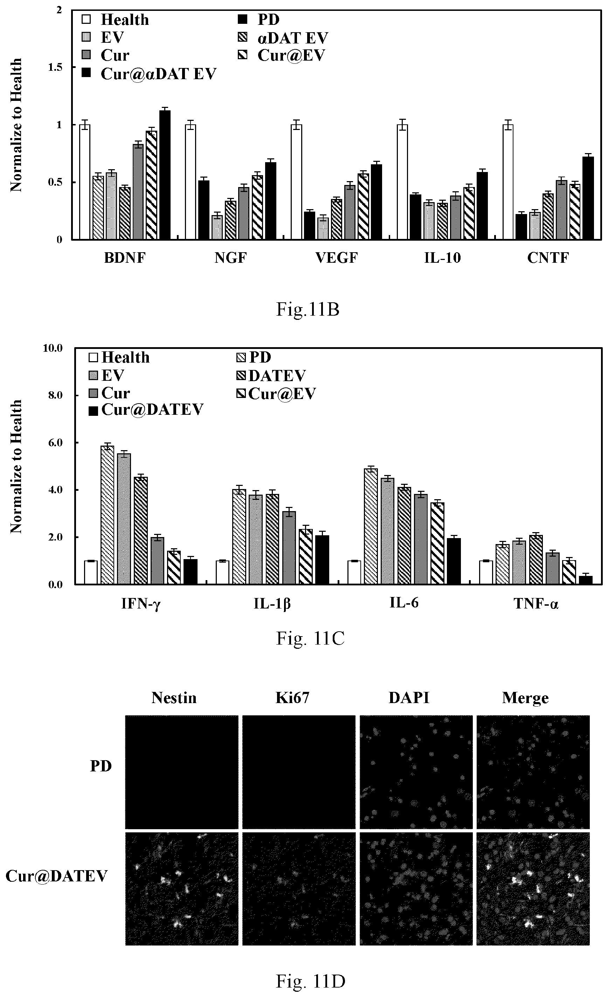

As shown in FIG. 11B, the experimental group demonstrates an upward trend in neurogenesis-related protein expression compared to the first through fifth control groups, particularly in BDNF, IL-10, and CNTF, with BDNF expression significantly exceeding that observed in the healthy control group.

As shown in FIG. 11C, inflammation-related protein expression in the experimental group demonstrates a downward trend compared to the first through fifth control groups, particularly for INF-γ, IL-1β, IL-6, and TNF-α, with TNF-α expression significantly lower than that observed in the healthy control group.

Referring to FIG. 11D, expression of neural stem cell-related proteins (nestin and Ki67) in dorsal striatum brain slices is compared between the first control group (PD) and the experimental group (Cur@DATEV) using immunofluorescence staining. The experimental group exhibits significantly higher expression levels of nestin and Ki67, thereby indicating that Cur@DATEV maintains neural stem cell activity within the dorsal striatum and promotes regeneration and differentiation of dopamine neurons.

Collectively, these results demonstrate that Cur@DATEV enables curcumin to traverse the blood-brain barrier, accumulate within the dorsal striatum, reduce alpha-synuclein secretion and accumulation, attenuate inflammation, decrease inflammation-related protein expression, enhance neurogenesis-related protein expression, and preserve neural stem cell activity, thereby promoting dopamine neuron regeneration and differentiation and improving motor function in the Parkinson's disease animal model.

### Experiment 5

Using the DATEV vehicle of the second embodiment, this experiment evaluates whether BDNF@DATEV can release mRNA-BDNF-CY3 into the Parkinson's disease model cell line 50 and effectively regulate BDNF expression. The Parkinson's disease animal model is treated by intravenous injection once per week from day 0 to week 5, and animals are assigned to the following groups:
- A healthy control group (Health), which undergoes sham surgery in the dorsal striatum brain area and receives no drug treatment;
- A first control group (PD), receiving no drug treatment;
- A second control group (BDNF@EV), treated with BDNF@EV vehicle;
- An experimental group (BDNF@DATEV), treated with BDNF@DATEV vehicle. Following phagocytosis by the Parkinson's disease model cell line 50, the release of mRNA-BDNF-CY3 and therapeutic efficacy are evaluated.

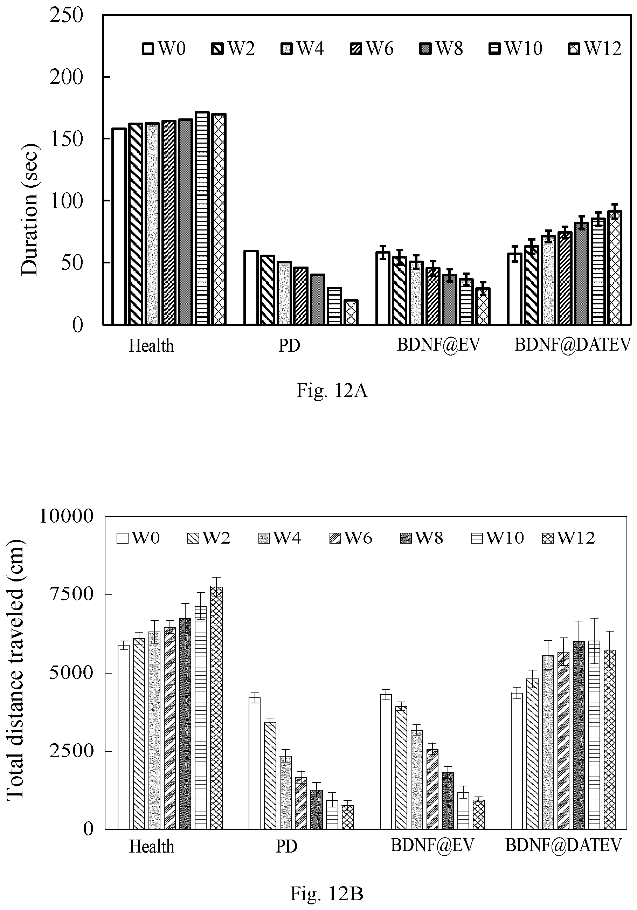

Referring to FIGS. 12A and 12B, the Parkinson's disease animal model in each of the groups described above is subjected to rotarod tests and open field tests beginning at week 0 and subsequently at weeks 2, 4, 6, 8, 10 and 12, respectively, to evaluate motor performance in each group. The assessments include measurement of rotarod duration and open field activity parameters.

The results show that motor performance in the first control group, second control group, and experimental group remains significantly lower than that of the healthy control group. However, motor performance in the experimental group progressively improves over time, with rotarod duration increasing by more than 60% and total distance traveled in the open field test increasing by more than 32% by week 12 compared to baseline values at week 0, thereby demonstrating that BDNF@DATEV treatment mitigates Parkinson's disease symptoms and restores behavioral function.

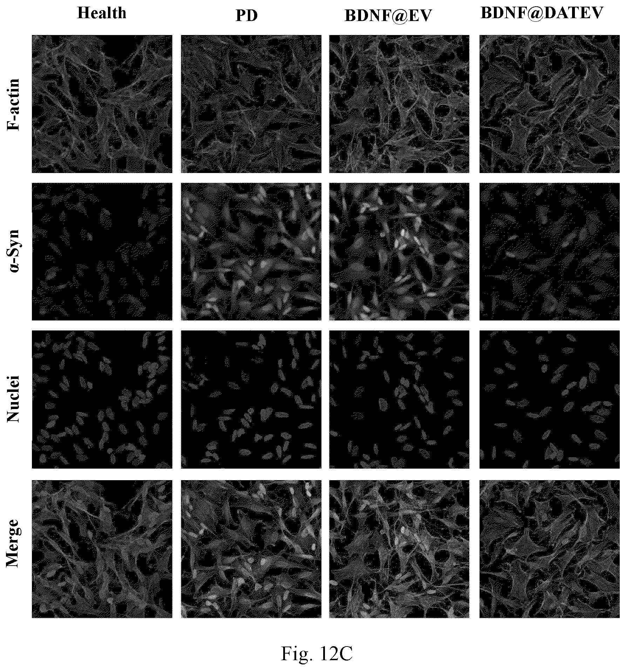

Referring to FIG. 12C, neural stem cell-related protein expression in dorsal striatum brain slices is analyzed by immunofluorescence staining, wherein F-actin is labeled with red fluorescence to visualize the cytoskeleton, nuclei are labeled with blue fluorescence to identify cellular nuclei, and alpha-synuclein is labeled with green fluorescence to visualize the accumulation of alpha-synuclein. The experimental group (BDNF@DATEV) exhibits significantly reduced alpha-synuclein accumulation relative to the second control group (BDNF@EV), consistent with the results obtained in the experiments of the first embodiment.

### Experiment 6

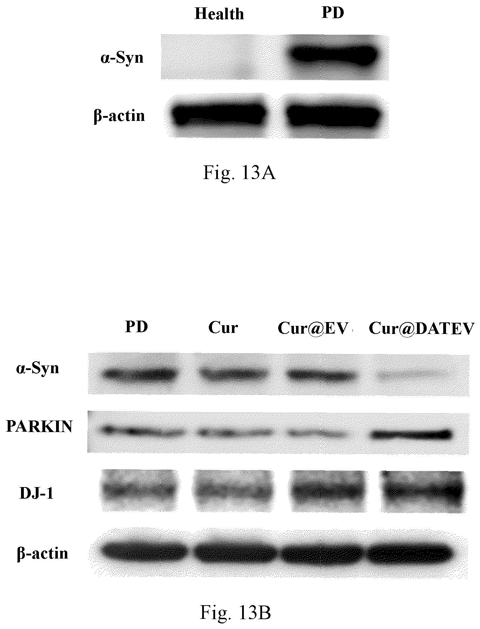

To confirm that the DATEV vehicle exerts similar therapeutic effects in human cells, induced pluripotent stem cells (iPSCs) derived from Parkinson's disease patients are differentiated into neurons (PD) for evaluation. As shown in FIG. 13A, Western blot analysis reveals that, relative to healthy control cells, the differentiated neuronal cells (PD) exhibit elevated expression of alpha-synuclein, thereby confirming that the iPSCs derived from Parkinson's disease patients were successfully differentiated into neuronal cells exhibiting the physiological protein biomarker profile characteristic of Parkinson's disease.

The differentiated neurons are subsequently treated with Cur@DATEV, and expression levels of alpha-synuclein, Parkin protein, and DJ-1 protein are analyzed on day 10 post-treatment. As shown in FIG. 13B, treatment with Cur@DATEV results in reduced alpha-synuclein expression and increased expression of Parkin protein and DJ-1 protein, findings that are consistent with results obtained from the prior experiments.

In FIGS. 2A and 2B, to verify that the targeting vehicle provided by the present invention may exhibit different capabilities according the difference of the parental cell line, the Parkinson's disease model cell line 50 was cultured in the blood-brain barrier cell culture model 10 to verify the therapeutic effectiveness of the third preferred embodiment.

### Experiment 7

The blood-brain barrier cell culture model 10 is treated under the following conditions:
- A first control group (PD), receiving no treatment;
- A second control group (EV), treated with the EV vehicle collecting from the HEK-293 cells;
- A third control group (MSCEV), treated with the MSCEV vehicle collecting from the MSC cells;
- A fourth control group (DATEV), treated with free curcumin;
- An experimental group (MSC-DATEV), treated with DATEV vehicle collecting from the DAT-MSC cell.

After the Parkinson's disease model cell line treated with the above groups for 24 hours, the endocytosis efficiency of the Parkinson's disease model cell line was confirmed. And confirming if the DATEV collected by DAT-MSC cells produce a therapeutic effect after the reaction for 48 hours.

The immunofluorescence staining and exosomal protein labeling technique (Protein EV Labeling Kit (Red), ExoGlow^{™}, System Biosciences, Palo Alto, CA, USA) was employed to labeling if the exosomal proteins are presented in the Parkinson's disease model cell line 50. And using the Flow cytometer to analyze the endocytosis efficiency. As shown in FIG. 16A, the Parkinson's disease model cell line 50 of the third control group (MSCEV) and the experimental group (MSC-DATEV) exhibits the higher endocytosis efficiency, comparing to the first, the second, and the fourth control group. These results show that the DATEV vehicle exhibits specificity toward the DAT protein and is capable of being internalized into the Parkinson's disease model cell line via phagocytosis. In particular, an intake proportion of the Parkinson's disease model cell line in the experimental group (MSC-DATEV) can be as high as more than 95%.

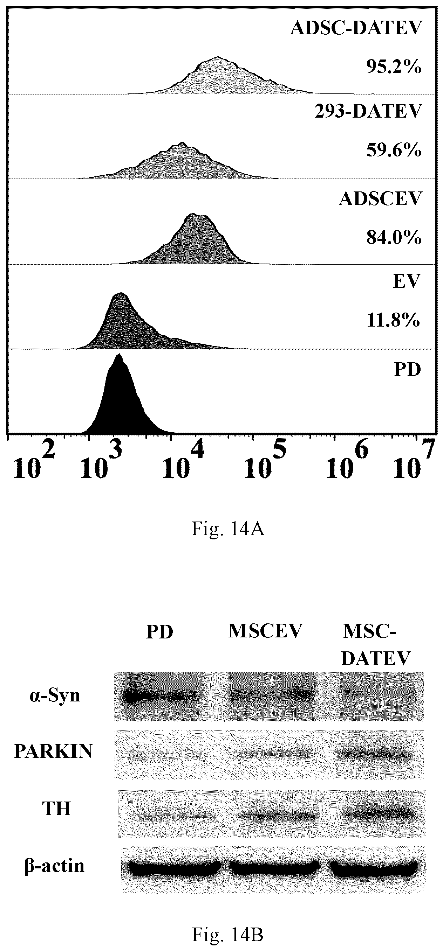

As shown in FIG. 14B, the first (PD), the third (MSCEV) control group and the experimental group (MSC-DATEV) are analyzed the expressions of alpha-synuclein, Parkin protein, and TH protein by Western blot after 48H treating. Worthy to note is that the experimental group (MSC-DATEV) can effectively reduce the expression of α-syn and increase the expression of Parkin protein and TH protein without any drug loading, which is great help in the development of cell repairing technology.

### Experiment 8

Further confirmed that if the DATEV vector obtained by collecting DAT-MSC cells can directly produce physiological effects on the Parkinson's disease animal model. Beginning on day 0 (i.e., the day of surgery), the Parkinson's disease animal model is treated weekly by intravenous injection for five consecutive weeks, and the animals are assigned to the following groups:
- A healthy control group (Health), which undergoes sham surgery in the dorsal striatum brain area and receives no drug treatment;
- A first control group (PD), which receives no drug treatment;
- A second control group (MSCEV), treated with the MSCEV vehicle collecting from the MSC cells;
- An experimental group (MSC-DATEV), treated with DATEV vehicle collecting from the DAT-MSC cell. The Parkinson's disease-related factor α-syn, neuroregeneration-related protein IL-10, and inflammatory response-related protein NF-γ in the dorsal striatum brain region and the motor performance of the Parkinson's disease animal model in each group were detected to confirm the physiological mechanism of the Parkinson's disease animal model after MSCEV vehicle treatment.

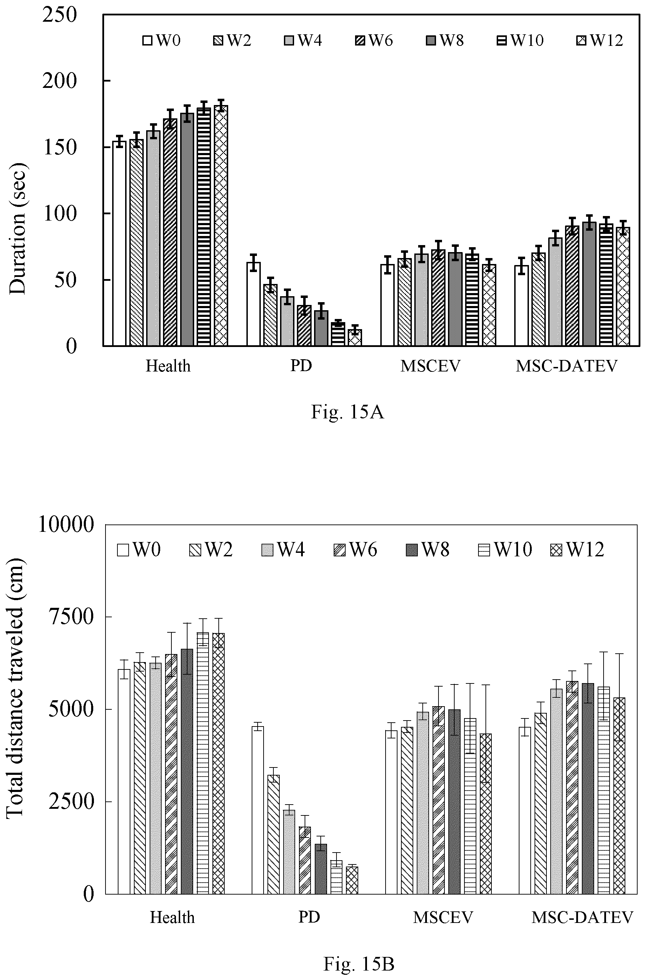

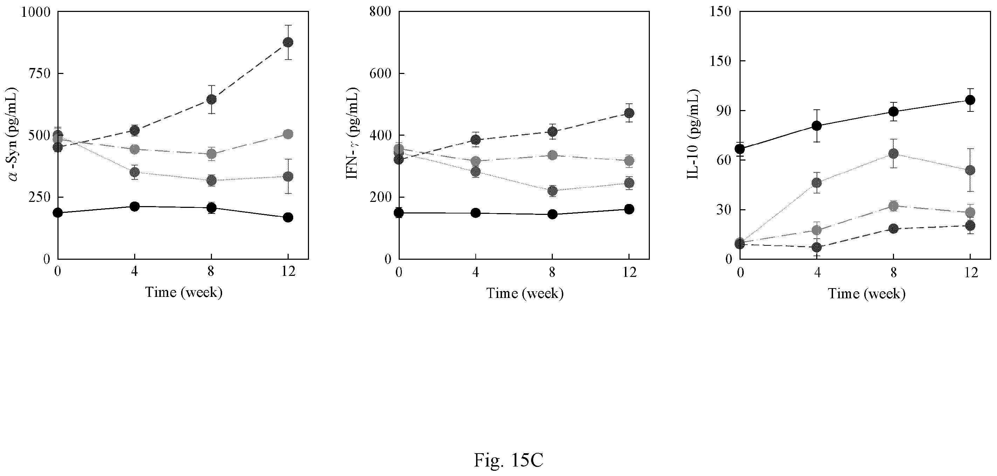

Referring to FIG. 15A and 15B, rotarod tests and open field tests are conducted at weeks 0, 2, 4, 6, 8, 10 and 12 to assess motor performance, including the time duration the rats are able to remain on the rotating rod, and measurements of movement speed, total distance traveled, and total rest time.

Whether in the results of the rotarod tests or the open field tests, it can be found that motor performance in first control group, second control group, and the experimental group is significantly impaired compared to the healthy control group.

However, only the experimental group (MSC-DATEV) exhibits progressive improvement in motor performance over time, especially at week 12, the duration of the rotarod tests of the experimental group increased by more than 48% compared with week 0, and the total distance traveled in the open field tests increased by more than 17% compared with week 0. This indicates that the experimental group (MSC-DATEV) treated with MSCEV effectively slowed down the symptoms of Parkinson's disease and even showed the effect of repairing the behavioral performance ability of the Parkinson's disease animal model.

As shown in FIG. 15B, the experimental group (MSC-DATEV) exhibits significantly reduced alpha-synuclein accumulation in the dorsal striatum relative to the first through fifth control groups. The expression of neuroregeneration-related protein IL-10 and inflammatory response-related protein NF-γ showed an upward trend, confirming that the extracellular vesicles secreted by DAT-MSC cells have the effect of directly regulating physiological mechanisms.

The DATEV vehicle provided by the present invention may be loaded with a drug to enable transport across the blood-brain barrier, achieve specific binding to dopamine neurons, regulate the expression of Parkinson's disease-associated marker proteins, and provide superior therapeutic efficacy in delaying the progression of Parkinson's disease.

Loading curcumin into the DATEV vehicle effectively reduces alpha-synuclein accumulation, decreases expression of inflammation-related proteins (INF-γ, IL-1β, IL-6, TNF-α), increases expression of Parkin protein, DJ-1 protein, TH protein, and neurogenesis-related proteins (BDNF, NGF, VEGF, IL-10, CNTF), and maintains neural stem cell activity (nestin and Ki67 proteins) within the dorsal striatum. Furthermore, the DATEV vehicle may also be loaded with BDNF mRNA to regulate BDNF expression, reduce alpha-synuclein accumulation, delay the onset and progression of Parkinson's disease symptoms, and promote dopamine neuron regeneration and differentiation, thereby demonstrating substantial therapeutic potential for the treatment of Parkinson's disease.

## Claims

1. A targeting vehicle comprising an extracellular vesicle, wherein a transmembrane protein of the extracellular vesicle is conjugated with a dopamine transporter antibody, wherein the extracellular vesicle is secreted by a cell transfected with a vector gene, and wherein at least a portion of the vector gene comprises SEQ ID NO: 1.

2. The targeting vehicle of claim 1, wherein the transmembrane protein comprises CD63, and wherein the dopamine transporter antibody is inserted into an extracellular loop between third and fourth transmembrane domains of the transmembrane protein.

3. The targeting vehicle of claim 1, wherein the cell comprises a human embryonic kidney 293 (HEK-293) cell or a mesenchymal stem cell (MSC).

4. The targeting vehicle of claim 1, wherein the transmembrane protein comprises CD63, and wherein at least a portion of CD63 comprises SEQ ID No:5.

5. The targeting vehicle of claim 1, wherein the transmembrane protein comprises CD63, and wherein at least a portion of CD63 comprises SEQ ID No:6.

6. A targeting vehicle comprising an extracellular vesicle, wherein a transmembrane protein of the extracellular vesicle is conjugated with a dopamine transporter antibody, wherein the extracellular vesicle is secreted by a cell transfected with a vector gene, and wherein at least a portion of the vector gene comprises SEQ ID NO: 6.

7. A pharmaceutical composition comprising the targeting vehicle of any one of claims 1 to 3, wherein the targeting vehicle is loaded with a drug, and wherein the drug comprises a gene fragment (DNA or RNA), a protein sequence, or a chemical agent.

8. The pharmaceutical composition of claim 7, wherein the extracellular vesicle is loaded with curcumin.

9. The pharmaceutical composition of claim 8 for its use for reducing alpha-synuclein accumulation in a striatum brain region of dopamine neurons undergoing degeneration or pathology.

10. The pharmaceutical composition of claim 8 for its use for increasing expression of Parkin protein, DJ-1 protein, and tyrosine hydroxylase (TH) protein in a striatum brain region of dopamine neurons undergoing degeneration or pathology.

11. The pharmaceutical composition of claim 8 for its use for increasing expression of neurogenesis-related proteins in blood of a subject having dopamine neuron degeneration or pathology in a striatum brain region, wherein the neurogenesis-related proteins comprise brain-derived neurotrophic factor (BDNF), nerve growth factor (NGF), vascular endothelial growth factor (VEGF), interleukin-10 (IL-10), and ciliary neurotrophic factor (CNTF).

12. The pharmaceutical composition of claim 8 for its use for reducing expression of inflammation-related proteins in blood of a subject having dopamine neuron degeneration or pathology in a striatum brain region, wherein the inflammation-related proteins comprise interferon-gamma (INF-γ), interleukin-1 beta (IL-1β), interleukin-6 (IL-6), and tumor necrosis factor-alpha (TNF-α).

13. The pharmaceutical composition of claim 8 for its use for increasing or maintaining neural stem cell activity in a striatum brain region of dopamine neurons undergoing degeneration or pathology.
